Europäisches Patentamt

European Patent Office

Office européen des brevets

(19)

(11) Publication number: **0 044 824**

A1

# EUROPEAN PATENT APPLICATION

(12)

(21) Application number: **81850089.4**

(22) Date of filing: **20.05.81**

(51) Int. Cl.³: **G 01 N 33/04**
**C 12 Q 1/04**

(30) Priority: **22.05.80 SE 8003859**

(43) Date of publication of application:
**27.01.82 Bulletin 82/4**

(84) Designated Contracting States:
**AT BE CH DE FR GB IT LI LU NL SE**

(71) Applicant: **AB Ferrosan**
**Celsiusgatan 35**
**S-201 10 Malmö(SE)**

(72) Inventor: **Petersson, Leif Ake Sigvard**
**Ormvräksgatan 42**
**S-214 62 Malmö(SE)**

(72) Inventor: **Willard, Lars-Olof**
**Mariedalsvägen 42**
**S-217 45 Malmö(SE)**

(74) Representative: **Bergvall, Stina-Lena et al,**
**Dr. Ludwig Brann Patentbyrä AB Box 7524 Kungsgatan 3**
**S-103 92 Stockholm(SE)**

(54) **Bacteriological test set for detecting butyric acid bacteria in milk.**

(57) Bacteriological testset for establishing the occurrence of butyric acid bacterial (*Clostridium*) in milk, comprising a sealed, by autoclaving sterilized vial containing a sterilized culture medium for anaerobic and microaerophilic cultivating, and an against the culture medium inert compound, solidifying at a temperature below about 40°C., having a density lower than the density of the culture medium, into which vial the milk to be tested is introduced under aseptic conditions; a method of establishing the occurrence of butyric acid bacteria in milk by adding the milk to be tested via a plastic drain into a sealed, autoclaved vial containing a sterilized culture medium for anaerobic and microaerophilic cultivating, and an against the medium inert compound, solidifying at a temperature below about 40°C., and having a density lower than the density of the culture medium; and the use of the testset for establishing the occurrence of butyric acid bacteria in milk by adding the milk to be tested through a plastic drain into a sealed, autoclaved vial containing a sterilized culture medium for anaerobic and microaerophilic cultivating, and an against the medium inert compound, solidifying at a temperature below about 40°C., and having a density lower than the density of the culture medium; whereafter the vial is heated and incubated and gas formation in the sample is read off.

EP 0 044 824 A1

AB Ferrosan, Sweden

1

Bacteriological testset for establishing butyric acid bacteria in milk.

The invention relates to a bacteriological testset for establishing butyric acid bacteria (Clostridium) in milk, comprising a sealed, through autoclaving sterilized vial containing a sterilized culture medium for anaerobic or microaerophilic cultivating, and an against the culture medium inert compound, solidifying at a temperature below about 40$^{O}$C. and having a density lower than the density of the culture medium, to which vial the milk to be tested is added under aseptic conditions; a method for establishing butyric acid bacteria in milk by adding the milk to be tested through a plastic drain into a sealed, autoclaved vial containing a sterilized culture medium for anaerobic and microaerophilic cultivating, and an against the culture medium inert compound solidifying at a temperature below about 40$^{O}$C. and having a density lower than that of the culture medium; and the use of the testset for establishing butyric acid bacteria in milk by adding the milk to be tested through a plastic drain into an autoclaved vial containing a sterilized culture medium for anaerobic or microaerophilic cultivating, and an against the culture medium inert compound solidifying at a temperature below about 40$^{O}$C. and having a lower density than that of the culture medium, whereupon the vial is heated and incubated and gas formation in the sample is read off.

Ensilage of inferior grade is generating butyric acid bacteria which with the fodder is transmitted to the cattle. Said butyric acid bacteria, consituting anaerobic, spore-forming bacteria (Clostridium), are transmitted to the milk. The spores of butyric acid bacteria survive pasteurization and form at the manufacturing of cheese except lactic acid also butyric acid and gas (hydrogen and carbon dioxide) - the cheese is going fermented. Therefore it is necessary to continuously check the occurrence of butyric acid bacteria in milk. Hitherto this has generally been made by a test known as Winzirl test in the following way:

1. Into a sterilized test-tube containing about 1 g. of paraffin 1 ml. of a glucose-lactic acid mixture and 10 ml. of the milk sample to be tested or an appropriate dilution thereof are added.

2. The test-tube with the milk sample is maintained for about ten minutes in a water bath at $80^{\circ}$C., whereby vegetative bacteria cells are killed and the air is expelled from the sample. The paraffin melts and settles on the milk surface and prevents the air to pass down into the milk.

3. The sample is incubated for 7 to 10 days at $30^{\circ}$C.

4. The occurrence of butyric acid bacteria is shown by the development of gas in the sample - the paraffin plug is lifted. The most likely number of butyric acid bacteria spores of the milk sample is then established with the aid of a special table.

Said method is expensive, work-demanding and even directly cumbersome when a large number of samples are to be taken. Therefore, a demand has existed for a simplified test system, easy to be handled to establish the presence of butyric acid bacteria in milk.

The demands on the new system are that it should be easy to handle, the culture medium has to be sterilized and the sterility maintained during prolonged periods of time, preferably several months and even years. It must be possible to shelve the culture medium without essential chemical
changes during a long period of time. The test system must be
easy to transport and shelve also after the inoculation, and
a clear reaction (gas formation) must also develop with very
low inoculates (<10 spores per ml. sample). The product must
also be cheap compared to the present handling.

Said problems have been solved with the test system according
to this invention, comprising a vial, sterilized by autoclaving
at 121$^{o}$C. for 15 minutes, containing a sterilized culture
medium for anaerobic or microaerophilic cultivating, such as
Reinforced Clostridial Medium (RCM) or another equivalent
culture medium, and one against the culture medium inert
compound, solidifying at a temperature below about 40$^{o}$C. having
a lower density than the culture medium, such as silicone or
preferably paraffin with a melting point of 56-58$^{o}$C. and
sealed with a rubber membrane and sheet metal.

The compound such as silicone or paraffin, inert against the
culture medium, should be used in an amount that allows the
gas to expand at the optional gas development occurring when
butyric acid bacteria are present at the test by pushing the
silicone or paraffin plug upwards by the gas pressure.

To facilitate the displacement of the paraffin or silicone plug
at the gas formation the vial should preferably be cylindrical
and the relation between the cross section diameter and the
height of the vial should be 1:5 to 1:2.5, preferably 1:3.
To further facilitate the handling and transport of the vial,
said vials can be packed standing upright in a durable wrapping
material of standard type.

The relation between the volume of the vial and the culture
medium should be 4:1 to 3:1, preferably 4:1, and between the
culture medium and paraffin or silicone 3:1 to 1:1, preferably
2:1.5.

The term "cylindrical" refers to an area generated by parallel displacement of a straight line along a closed curve.

The invention also relates to a method for establishing butyric acid bacteria in milk, according to which method the milk to be tested through a disposable plastic drain is added to the sealed vial sterilized by autoclaving at 121$^{\text{o}}$C. for 15 minutes through the rubber membrane, whereafter the vial with the drain is placed into a water bath at 80$^{\text{o}}$C. for 10 minutes and then incubated at 31$^{\text{o}}$C. for 4 to 7 days, and the gas formation of the sample is subsequently read off.

Finally the invention relates to the use of the testset as hereinbefore defined to establish butyric acid bacteric in milk. The culture media and materials to be used are preferably:

Reinforced Clostridial Medium (RCM)  CM 149, OXIDE
Paraffin, m.p. 56 to 58$^{\text{o}}$C.  No. 1.3643, KEBO
Oxford syringe
Plastic drain (disposable) 1 ml.  OXFORD LABORATORIES
Vial,4 to 10 ml., sheet metal, rubber
membrane

Example.
6.5 ml. vials were filled with 1.1/2 ml. melted paraffin. When solidified, the vials were further filled with 2 ml. RCM. Thereafter the vials were sealed with sheet metal and rubber membrane and autoclaved for 15 minutes at 121$^{\text{o}}$C. The autoclaved vials were packed standing upright in a durable wrapping material of standard type and transported to the user.

The paraffin and RCM filling can be performed in one or two steps.

To establish the presence of butyric acid bacteria spores in the sealed and autoclaved vials containing culture medium and paraffin, about 1 ml. milk samples are added through the rubber membrane by a sterilized disposable plastic drain, whereafter the vials with drains were placed in a water bath at 80$^{\text{o}}$C. for

10 minutes and finally transferred to the incubator, where incubation is performed at 31°C. for 4 to 7 days.

The culture medium was shown to be durable up to six months without deterioration of the function in any respect. It is true that a certain turbidity can appear after some time in the culture medium, but this disappears at heating. The wrapping as such as well as the sealing stand transport as well as storage. The drain can be removed when reaction is positive (gas formation), and the culture can be transferred to special laboratories for indentification purposes.

To control the occurrence of butyric acid bacteria spores in milk, 1 ml. milk is added by Oxford syringe and disposable plastic drain. The drain is left in the rubber membrane. The vial is heated for 10 minutes at 80°C. in a water bath to kill vegetative cells. The paraffin is melted. The sample is incubated for 4 to 7 days at 31°C. The paraffin is melted again and settles on the mixture of culture medium and milk sample.

After incubation the sample is read as to gas formation. The paraffin plug is pushed upwards in the vial and indicates in that way the occurrence of butyric acid bacteria in the sample in the case gas formation is occurring. A sample without butyric acid bacteria does not show any gas formation.

The invention is further elucidated with reference to the enclosed drawing wherein

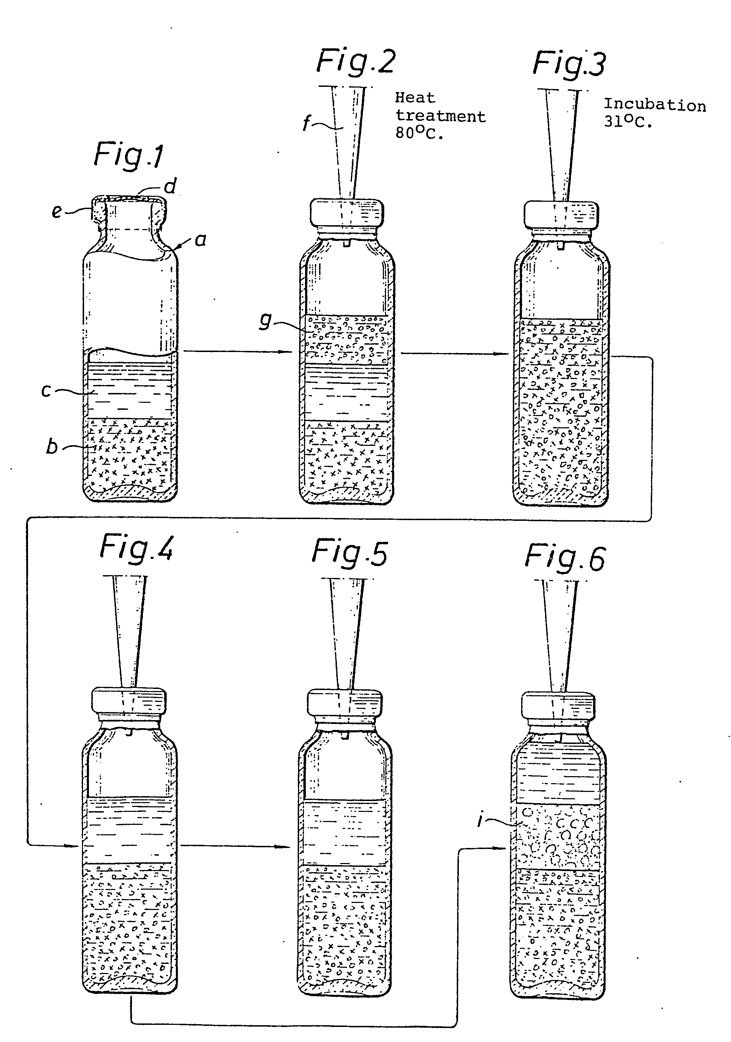

Fig. 1    shows a vial (a) containing a culture medium (b) and
          paraffin (c) and sealed by a rubber membrane (d) and
          sheet metal (e). The vial is autoclaved for 15 minutes
          at 121°C. The vial is packed in standard wrapping and
          ready for supply.

Fig. 2    shows the vial 1 containing the milk sample (g) added
          through a syringe and plastic drain (f) through the
          rubber membrane. The drain is left in the rubber membrane.

Fig. 3   shows the vial heated for 10 minutes at 80°C. in a
         water bath to kill vegetative cells. The paraffin is
         melted.

Fig. 4   shows the vial after incubation for 4 to 7 days at
         31°C. The paraffin has solified again and settled on
         top of the mixture of culture medium and sample.

Fig. 5   shows a vial containing a sample without butyric acid
         bacteria, which does not give any gas formation.

Fig. 6   shows gas formation (i) after incubation. The paraffin
         plug is pushed upwards in the vial, indicating the
         occurrence of butyric acid bacteria in the original
         sample.

CLAIMS:

1.    Testset for establishing the occurrence of butyric acid
bacteria in milk, comprising a sealed, through autoclaving
sterilized cylindrical vial containing a sterilized culture
medium for anaerobic or microaerophilic cultivating, and a
compound inert against the culture medium, solidifying at
a temperature below about $40^{\circ}$C., having a density lower than
the density of the culture medium, to which vial the milk to
be tested is added under aseptic conditions.

2.    Testset according to claim 1, characterized in that
the sterilized culture medium is Reinforced Clostridial
Medium (RCM) or another similar medium, and that the compound
inert against the culture medium is silicone or paraffin
having a melting point of 56 - $58^{\circ}$C.

3.    Testset according to claim 1, characterized in that
the vial has substantially parallel walls, that the ratio
between the cross section diameter and height of the vial is
from 1:5 to 1:2.5, preferably 1:3, and that the ratio between
the volume of the vial and the volume of the culture medium plus
compound inert against the culture medium is from 2:1 to
1.5:1, preferably 2:1.

4.    Testset according to claim 1, characterized in that
the ratio between the culture medium and the compound inert
against the culture medium is from 3:1 to 1:1, preferably 2:1.5.

5.    A method of establishing the occurrence of butyric acid
bacteria in milk, characterized in that the milk to be tested
is added to a sealed, by autoclaving at $121^{\circ}$C. for 15 minutes
sterilized vial containing a sterilized culture medium and an
against the culture medium inert compound, solidifying at at
temperature below about $40^{\circ}$C., having a density lower than the
density of the culture medium.

6.    A method according to claim 1, characterized in that the milk is added into the vial, autoclaved at 121°C. for
15 minutes and sealed by a rubber membrane and sheet metal,
by a sterilized    disposable plastic drain through the rubber
membrane, and that the vial with the drain is placed in a water
bath at 80°C. for 10 minutes and then incubated at 31°C. for
4 to 7 days.

7.    A method according to claim 5, characterized in that
the ratio between culture medium and milk is from 4:1 to 2:1,
preferably about 2:1.

8.    A method according to claim 5, characterized in that
after the incubation the gas formation of the sample developed
by butyric acid bacteria is read off.

9.    The use of a testset to establish the occurrence of
butyric acid bacteria in milk, characterized in that the milk
to be tested is added into a sealed, by autoclaving sterilized
vial containing a sterilized culture medium for anaerobic or
microaerophilic cultivating, and an against the culture medium
inert compound solidifying at a temperature below about 40°C.,
having a lower density than the culture medium, whereafter
the vial is heated and incubated and the optional gas formation
of the sample is read off.

10.    The use of a testset according to claim 9, characterized
in that to the vial, sealed by a rubber membrane and sheet metal,
containing a sterilized culture medium for anaerobic and
microaerophilic cultivating, such as Reinforced Clostridial
Medium, and a compound inert against the culture medium, which compound solidifies at a temperature below about 40°C., has a lower
density than the culture medium, such as silicone or preferably
paraffin, and a melting point of 56 - 58°C, the ratio between
the volume of the vial and the culture medium plus the compound
inert against the medium compound being from 2:1 to 1.5:1, preferably
2:1, and the ratio between the culture medium and the inert compound
being from 3:1 to 1:1, preferably 2:1.5, milk is added in an
amount corresponding to the ratio 1:4 to 1:2, preferably about 1:2, calculated on the amount culture medium as used, through a disposable plastic drain, the vial being heated at 80$^{\circ}$C. for 10 minutes, incubated at 31$^{\circ}$C. for 4 to 7 days and the gas formation in the sample developed by butyric acid bacteria read off.

_Fig.2_

Heat
treatment
80°C.

_f_

_Fig.3_

Incubation
31°C.

_Fig.1_

_d_

_e_

_a_

_c_

_b_

_g_

_Fig.4_

_Fig.5_

_Fig.6_

_i_

**0044824**

Application number

**EP 81 85 0089**

# European Patent Office

## EUROPEAN SEARCH REPORT

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | G 01 N 33/04<br>C 12 Q 1/04 |
| A | MICROBIOLOGY ABSTRACTS, Section A, Industrial & applied microbiology vol.13, no.12, December 1978 page 173, column 2, abstract no. 9458 LONDON (GB) & Med. Microbiol. Immunol. 165(1), 67-72, 1978 B.L. LINDQUIST et al.:"Use of a rapid fermentation test for identification of anaerobic bacteria" | | |
| | -- | | **TECHNICAL FIELDS SEARCHED (Int. Cl.³)** |
| A | BIOLOGICAL ABSTRACTS, vol.70, 1980, page 3150, column 1, abstract no. 29969 PHILADELPHIA (US) & S Afr. J. Dairy Technol. 11(3) 125-132, 1979 J.F. MOSTERT et al.:"Isolation, identification and practical properties of Bacillus spores from ultrahigh temperature and sterilized milk" | | G 01 N 33/00<br>33/04<br>33/16<br>C 12 Q 1/02<br>1/04<br>1/06<br>B 01 L 3/14 |
| | -- | | |
| A | BIOLOGICAL ABSTRACTS, vol.67, no.4, 1979, page 2130, column 1, abstract no. 21651 PHILADELPHIA (US) & Appl Environ Microbiol 36(4) 567-571, 1978 J.E. ERICKSON et al.:"New medium for rapid screening and enumeration of Clostridium perfringens in foods" | | **CATEGORY OF CITED DOCUMENTS**<br><br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| | -- ./. | | |
| | The present search report has been drawn up for all claims | | &. member of the same patent family, corresponding document |

| Place of search | Date of completion of the search | Examiner |
|---|---|---|
| The Hague | 04-09-1981 | GRIFFITH |

EPO Form 1503.1 06.78

0044824

European Patent Office

**EUROPEAN SEARCH REPORT**

Application number

EP 81 85 0089

-2-

| | DOCUMENTS CONSIDERED TO BE RELEVANT | | CLASSIFICATION OF THE APPLICATION (Int. Cl.3) |
|---|---|---|---|
| Category | Citation of document with indication, where appropriate, of relevant passages | Relevant to claim | |
| A | CHEMICAL ABSTRACTS, vol.72, no.13, March 30, 1970, page 258, column 1, abstract no. 65480n COLUMBUS, Ohio (US) & Lait. 1969, 49(488), 507-19. J.L. BERGERE et al.:"Sterilization of milk and removal of Clostridium tyrobutyricum spores" | | |
| | -- | | |
| A | US - A - 3 553 082 (C.C. HACH) | | TECHNICAL FIELDS SEARCHED (Int. Cl 3) |
| | ------- | | |

EPO Form 1503.2   06.78